# EUROPEAN PATENT APPLICATION

(11) **EP 2 145 617 A1**
(43) Date of publication of application: **20.01.2010**
(21) Application number: 09005055.0
(22) Date of filing: 06.04.2009
(51) Int. Cl.: A61K 9/06, A61K 31/191, A61K 33/06, A61P 17/02

(54) **Gel formulation**

(30) Priority: 04.04.2008 GB 0806119
(71) Applicant: Ludzker, Benjamin, Liverpool L19 3PG (GB)
(72) Inventor: Ludzker, David, Liverpool L6 1HH (GB)
(74) Representative: McDonald, Christopher Ian

(57) **Abstract**

The present invention relates to a sterile gel formulation comprising calcium gluconate. It also relates to a sterilisable gel formulation comprising calcium gluconate, which is stable under gamma-radiation sterilisation conditions, and also to the use of these formulations in, for example, treating burns caused by exposure of a human or animal body to hydrofluoric acid, either in liquid or vapour form.

## Description

The present invention relates to a sterile gel formulation comprising calcium gluconate, and to a sterilisable gel formulation comprising calcium gluconate, which is stable under gamma-radiation sterilisation conditions. These formulations are useful, for example, in treating burns caused by exposure of a human or animal body to hydrofluoric acid, either in liquid or vapour form..

Hydrofluoric acid, HF, is one of the strongest known inorganic acids. It can cause serious burns, and even death, upon sufficient contact with a human or animal body.

Despite the hazards associated with it, HF is a versatile and widely used chemical. It is used in the industrial synthesis of fluorine-containing compounds such as Teflon and refrigerants, it is used in the semiconductor industry, in etching glass, in oil refineries, and in purifying stainless steel, aluminium and uranium. Dilute HF is now sold as rust stain remover and has even been used in wheel cleaner compounds for washing cars. Reports of HF burns have therefore become more commonplace.

The dangers arising from HF spills have been recognised for a long time, being first documented in 1809. A considerable amount of research has been carried out since then to devise the optimum mode of treatment for HF burns.

Most HF burns are to the bodily extremities such as the hands and arms, and are caused by dilute aqueous HF solutions. However, industrial HF accidents often involve more concentrated HF solutions or HF gas and can cause life-threatening injuries.

Upon contact with a human or animal body, HF produces an initial corrosive burn on the skin. This is the case with all strong inorganic acids, and is due to the rapid release of hydrogen ions. The damage caused by the acid is directly related to the concentration of the acid and the duration of contact with the human or animal body. Thereafter, however, the mode of action of HF is different to other inorganic acids, and significantly more harmful.

It is the secondary damage HF causes which differentiates it from the other inorganic acids and which requires urgent treatment to prevent extensive and potentially long-term damage. This is because of the ability of the fluoride ion to penetrate through all layers of the epidermis, dermis, and even deeper subcutaneous tissues. This can result in severe destruction, liquefaction necrosis, and even injuries to underlying bone due to decalcification as the fluoride ions react readily with Ca²⁺ ions present in the bone.

It is this property of HF that makes exposure to it especially dangerous and the injuries it causes very difficult to treat. This secondary process can go undetected and, if untreated, can continue for several days, causing increasing tissue damage and, in time, systemic fluorosis.

The mechanism of injuries caused by HF burns is attributed to the reaction of the fluoride ions with the biologically important Ca²⁺ and Mg²⁺ ions to form insoluble salts. These salts then interfere with cellular metabolism, which can lead to cell death and necrosis.

A further unusual characteristic of HF burns is that, depending on the concentration of the acid, there often is a delay in the onset of tissue damage. That is, very dilute acids do not produce any changes in the appearance of the skin until a number of hours have elapsed. This can create difficulties in determining the time and origin of the burn, or even in a person realising until some time after the event that they have been exposed to a source of HF.

If the HF is up to about 20% in concentration, it can take up to 24 hours for a burn to be clinically evident, and from 20-50% it will take approximately from 1-8 hours. If it is upwards of 50% in concentration, it will take less than an hour, and for HF in a concentration of about 99% the damage will be immediate.

An intense pain is experienced with HF burns when the damage it causes becomes evident. This is thought to be caused by irritation of nerve endings by increased levels of K⁺ ions entering extracellular tissues to compensate for the reduced levels of Ca²⁺ ions precipitated by fluoride ions. Relief of this pain is an important guide to the success of any treatment.

The initial signs of an HF burn are redness, edema, or blistering and, with more concentrated acids, a blanched white appearance. The fluoride ion then penetrates the upper layers of the skin, leading to liquefaction necrosis of the deeper tissues, with a creamy yellow precipitate forming under the raised skin. The next stage is decalcification and corrosion of underlying bone.

Significant negative systemic effects can occur, the most serious being due to the scavenging of Ca²⁺ and Mg²⁺ ions by the fluoride ion. This produces serious adverse effects on cardiac function and nerve conduction and leads to systemic fluorosis. As little as 7 ml of anhydrous HF (*i*.*e.* HF at a concentration of >99%) in contact with the skin and untreated can bind all the free calcium ions in a normal-size adult male. Indeed, with burns involving greater than 25 square inches (161.3 cm²), significant, even fatal, hypocalcaemia can result.

The site of the burn is also important. Facial burns allow for much more rapid absorption of fluoride ions due to the increased vascularity of that area compared with the rest of the body, and also create the risk of inhalation injuries. A fatality has been reported due to a facial burn covering only 2.5% of the body but producing significant hypocalcaemia and cardiac arrest. A number of deaths have also been due to pulmonary edema from inhalation of HF fumes. Severe burns to the mucous membranes may also occur from ingestion or inhalation.

When treating HF burns, the intention is to scavenge all of the fluoride ions which have been introduced into the body. There are a number of methods currently used to achieve this. One is to soak the burn area with an iced aqueous or alcoholic solution of a high molecular weight quaternary amine such as Hyamine 1622 (benzethonium chloride) or Zephiran (benzalkonium chloride).

Another method is to use Ca²⁺ ions to which the fluoride ions readily bind. An insoluble salt is formed so no further tissue damage can follow. Calcium gluconate is the typical source of the Ca²⁺ ions. The calcium gluconate can be administered either by injection or in a gel form. The gel form of the calcium gluconate typically comes as a 2.5% concentration of calcium gluconate in a water-soluble lubricant.

Following decontamination and copious washing, the gel acts as a reservoir of Ca²⁺ ions to draw out the fluoride ions from the damaged tissue. The gel is massaged into the damaged skin repeatedly over a long period of time, usually several hours and sometimes as much as 3 to 4 days, four to six times per day. Once most or all of the Ca²⁺ ions in the gel have reacted with the fluoride ions, the used gel can be replaced with further quantities of fresh gel to continue the process.

As there is such a discrepancy between the concentration of Ca²⁺ ions in the gel on the surface of the damaged skin and the underlying tissue, a concentration gradient forms between them. This gradient promotes the movement of the fluoride ions from the underlying tissue through the upper layers of the skin to the surface to react with the Ca²⁺ ions in the gel formulation.

The advantages of this method are the convenience of using a gel and the simplicity of treatment, although existing calcium gluconate gels cannot be safely applied to damaged skin as they are non-sterile.

New proposed EU regulations mean that any product used on damaged skin must be sterile. Sterilisation of a material is typically carried out by irradiating it, for example with gamma rays, or exposing it to heat treatment. However, despite the normal stability of the currently available calcium gluconate gel formulations (they are stable for storage for 3 years), previous attempts to sterilise them have shown many of them to be unstable after exposure to irradiation, causing them to lose viscosity and break down. This, of course, renders them useless for treatment.

The problem with the formulation of a gel capable of being sterilised is the inclusion of the calcium gluconate as its ionic nature disrupts the gel formation and interferes with its rheological characteristics. An alternative to the current cellulose derived gelling agents is generically known as a carbomer. This is the basis for many commercial gels and consists of a C=C double bond which binds to the water to form a stable gel. Experiments with this type of compound have shown that exposure to irradiation causes the gel to reform and, in the gels used, to change the rheological characteristics into a thicker gel. It can also have the effect of complexing the Ca²⁺ ions into the reformed gel. This complexation in turn significantly affects the ability of the gel to treat HF burns as the release of the Ca²⁺ ions from the gel is inhibited, with the result that the Ca²⁺ ions cannot then scavenge for the F⁻ ions.

There are only a few sterile gels in existence for any purpose. In EP 693292, an amount of >15% w/v of a polyhydric alcohol is required to stabilise an alginate gel against a loss in viscosity during sterilisation. US 6,251,423 details how the presence of a hydrophilic cellulose derivative in water forms a gel which stabilises an emulsion during sterilisation. There are also documents describing the production of non-sterile calcium gluconate gels for the treatment of hydrofluoric acid burns, such as JP 63104914 and JP 64034915. There are however no calcium gluconate gel formulations available which can be exposed to conditions of sterilisation and remain effective in the treatment of HF burns.

According to the present invention there is therefore provided a sterile gel formulation comprising calcium gluconate.

There is also provided in accordance with the invention a sterilisable gel formulation comprising calcium gluconate, wherein the formulation is stable under gamma-radiation sterilisation conditions. By "stable under gamma-radiation sterilisation conditions" is meant that the gel formulation substantially retains its structure and remains effective in the treatment of HF burns. Once sterilised, the gel formulation can be used to treat HF burns on a physiological target site in accordance with the new proposed EU regulations.

Also provided by the present invention is a sterile gel formulation which is formed once the calcium gluconate sterilisable gel has been exposed to gamma-radiation sterilising conditions and sterilised. Typically, the level of radiation used may be from 10 kGy up to 25 kGy.

The formulation also comprises a gelling agent selected from pharmaceutical grade components. Typical polymers are super-absorbent polymers such as those disclosed in WO 92/16245 and comprise hydrophilic cellulose derivatives which have been partially cross-linked to form a three dimensional structure. Suitable compounds include those of the carboxy and hydroxyalkyl celluloses, wherein the alkyl group contains from 1 to 6 carbon atoms. A typical polymer for use in the invention is a partially cross-linked sodium carboxymethylcellulose of a suitable pharmacological grade. This polymer is a superabsorbent polymer in that it may absorb at least about ten times its own weight of water. The cross-linked structure of the polymer prevents it from dissolving in water but water is easily absorbed into and held within the three-dimensional structure of the polymer to form a gel. Water is lost less rapidly from such a gel than from a solution and this is advantageous in slowing or preventing the drying out of the gel formulation.

In addition to sodium carboxymethylcellulose, hydroxyethylcellulose, carbomer powder and xanthan gum can advantageously be used as the gelling agent.

In the embodiment of the invention where the gel formulation is stable under gamma-radiation sterilisation conditions, the gelling agent is typically a pharmaceutical grade carbomer powder.

The gelling agent is typically present in an amount of between about 0.8 and about 6.0% by weight, more typically between about 1.5 and about 4.0%, still more typically between about 2.0 and about 3.5%, still more typically between about 2.5% and 3.3%, and most typically about 3.0%.

The active calcium gluconate component is typically present in the gel formulations in an amount of between about 1.0 and about 5.0% by weight, more typically between about 1.5 and about 4.0%, still more typically between about 2.0 and about 3.5%, still more typically between about 2.0% and 3.0%, and most typically about 2.5%.

A quantity of a pharmaceutically acceptable alkali solution is also typically included in the gel formulation of the invention to adjust its pH. If the gel is a water-based gel, the alkali solution is typically a solution of NaOH. The NaOH solution will typically have a concentration of between about 8-12%, more typically about 10%.

If the gel is an alcohol-based gel, the alkali solution typically comprises triethanolamine in the same concentration levels as the NaOH.

It will be appreciated that any pharmaceutically acceptable alkali solution may be used to adjust the pH of the formulation to a desired level, and that the alkali solution will be tailored to water- and alcohol-based gels. It will also be appreciated that as much or as little alkali solution may be added as is required to achieve the desired pH, depending upon the pH of the formulation prior to addition of any of the alkali solution and the concentration of the alkali solution.

The desired pH of the calcium gluconate gel formulation is typically between about 5.0 and about 8.0, more typically between about 5.5 and about 7.5, still more typically between about 6.5 and about 7.5, most typically about 7.0.

Typically, the pH of the gel formulation before any alkali solution is added is about 5.5 to about 6.5. After addition of the alkali solution, the pH is typically about 6.5 to about 7.5.

The sterile calcium gluconate gel formulation, and the sterilisable calcium gluconate gel formulation of the invention (once sterilised), can be safely applied to damaged skin.

Water, typically de-ionised, makes up the total weight of the formulation to 100%.

Further optional components which may be added to the formulation of the invention include, but are not limited to, components which are conventionally used in existing gel formulations, such as but not limited to *e*.*g*. a preservative and/or a humectant.

The physiological target site may be any site of an HF burn on the body of a human or animal, but it will typically be on a human.

According to a further aspect of the invention, there is provided a method of manufacturing a sterile gel formulation comprising calcium gluconate, comprising the steps of:
i) substantially dissolving a quantity of calcium gluconate in an aqueous or alcohol-based solution;
ii) adding a gelling agent to the solution;
iii) mixing the solution until it is substantially homogeneous;
iv) if necessary, adjusting the pH to between about 5.0 and about 8.0;
   and
v) sterilising the gel.

Suitable methods of sterilising the formulation include, by way of example, heat/steam treatment, and aseptic production.

Of course, it will be appreciated that the method of preparing the sterilisable calcium gluconate gel formulation is stable under gamma-radiation sterilisation conditions, prior to the gamma-radiation sterilisation, can be the same as the method detailed under steps (i)-(iv) above.

Typically, the aqueous solution will be de-ionised water, and the pH will typically be adjusted using a pharmaceutically acceptable alkali solution, often an about 10% solution of NaOH.

The gelling agent used in step (ii) is typically a pharmaceutical grade carbomer powder, but may also be sodium carboxymethylcellulose, hydroxyethylcellulose, or xanthan gum.

By "substantially dissolving" it is meant that at least 97% of the calcium gluconate has been dissolved in the aqueous solution, or where the aqueous solution has become saturated with dissolved calcium gluconate.

By "substantially homogeneous" it is meant that the mixture is mixed until it appears to be primarily composed of one liquid phase.

Gamma radiation and steam sterilisation methods may be used to sterilise the gel once it has been packed into suitable packaging such as tubes, sachets, sealed pots or tubs. The essential requirement for this packaging is that it is able to withstand the conditions of sterilisation and retain a sterile barrier for the shelf life of the product.

Conventionally, when sterilisation is carried out using using gamma-radiation, the irradiation is conducted using a full 25 kGy dose. It is widely accepted that this level of irradiation is enough to kill any microbiological contamination and completely sterilise formulations. While using reduced dosages, such as 10, 15 or 20 kGy may provide a sterile product, one cannot be certain of the fact. Therefore, tests and extensive monitoring must then be carried out to verify the sterility of the product. The burden of proving the sterility is not as onerous after using a full 25 kGy dose.

The sterilisable gel formulation of the invention is the only one which is stable under exposure to the full 25kGy gamma irradiation dose, and which remains effective for the treatment of HF burns thereafter. All other formulations degrade and lose their structure and viscosity and are no longer fit for their intended purpose.

The use of steam autoclaves for sterilisation presents other problems to the production process: sterility assurance, packaging type, format and design.

Conventionally, steam sterilisation occurs by introducing high temperature steam into the product through its packaging which is deliberately porous, the steam permeates through the product and raises the temperature sufficiently high and for long enough to kill the microbiological contamination. This direct method cannot be used where the product is in a sealed waterproof container. In this instance steam must be generated within the product by heat transfer from the steam outside the packaging, a natural physical process if the temperature is held for long enough.

The conditions associated with sterilisation using steam are somewhat extreme. Typically, temperatures of up to about 130°C are reached, coupled with elevated pressures of potentially up to about 2-3 bar, for about 15 minutes.

To form steam in the packaging there must be a presence of 'free water' in the gel structure in the region of at least about 5%. This will affect the type of gel structure and rheology of the final product.

The free water in the gel boils under the conditions of sterilisation and creates steam which kills the undesirable microbiological contamination in the formulation. If there is insufficient free water in the formulation, this process cannot happen and the sterility cannot be guaranteed.

Furthermore, existing gel formulations typically contain an amount of a humectant, typically between about 10-40% of the overall formulation. A humectant is an extremely hygroscopic substance which absorbs and bonds to the water which is present in the gels. This water is thus not 'free' as it is bound to the humectant via hydrogen bonds. It is therefore not available to be boiled and transformed into steam, and so the steam sterilisation process cannot be successfully carried out on such formulations.

Therefore, according to another aspect of the invention, there is provided a sterilisable gel formulation comprising calcium gluconate which comprises no more than about 7% by weight, more typically no more than about 5%, of a humectant, or even no humectant at all. There is also provided a sterilisable gel formulation which comprises at least about 5% by weight of free water to enable the sterilisation process to be carried out.

As well as the elevated temperature considerations, the generation of the steam also acts to increase the pressure in the packaging. Under such conditions, conventional packaging for the gels, which is typically a low melting point polyolefin such as *e*.*g*. polyethylene, either melts, decomposes or splits, and releases its contents. It is therefore unsuitable for acting as a container for the gel for its sterilisation under these conditions. Therefore, the packaging has to be tailored to allow for the internal formation of steam while resisting the forces produced by the formation of the steam.

A conventional aluminium based tube has been used in the past with some success but aluminium is expensive and prone to permanent distortion.

The high temperatures involved in the process preclude the use of the traditional packaging favourite low melting point polyolefins. Alternative packaging is thus required which can withstand these sterilisation conditions. There are a range of film types that can be used, such as a three- or four-side sachet made from coextruded films or laminated films that may contain specific barrier layers to retard oxygen or moisture permeation. Examples of films include polypropylene/polyester, nylon/polypropylene. Alternatively, laminates containing low oxygen or moisture permeation films such as ethyl vinyl alcohol.

A balance needs to be struck for the packaging to fill ratio for a given gel. Too high of a fill quantity produced too much internal pressure upon steam generation and damaged the packaging. It was necessary to give sufficient internal volume to the packaging to allow for the steam expansion during sterilisation. This is dependent on the quantity and free water content of the gel, which is further dependent on the gelling agent used.

An essential part of the treatment of an HF burn with a calcium gluconate gel is to ensure that the gel is applied liberally to the affected area. Ostensibly, this is quite a simple requirement and should require little skill. However, the gel is substantially colourless. This makes it difficult to spread a thick, even layer of gel as a person cannot see the gel clearly to ascertain which parts of the gel layer are insufficiently thick. The parts of the layer which are insufficiently thick result in a treatment deficiency for the areas of the skin thereunder, so these areas will not be healed as quickly as the others.

According to one aspect of the invention, a trace colour may be included in the gel formulation. This allows a person applying the gel to see it more clearly and thus be able to estimate the relative thickness of the layer and achieve a more even application of the gel. The trace colour would essentially give a contrast to the skin colour and allow the visual determination of the thickness of the layer. While any colour could be used, colours such as blue or green are particularly suitable as they give an unnatural colour to the skin tone. The source of the colour can be from many chemical groups, but in general it would be a water soluble colorant which has very low skin toxicity and which is stable in the presence of the gel. A food dye, for example, could be used.

According to another embodiment of the invention, the formulation may comprise a physiologically acceptable indicator substance. Such an indicator is to show the presence of the fluoride or the acid formed. As the fluoride ions are scavenged by the calcium ions, the colour of the gel changes to indicate the presence of fluoride ions. This colour change thus indicates when the gel is becoming exhausted in specific areas and the calcium levels have become depleted, specifically where the fluoride concentrations are at their highest. This colour change indicates to a person that fresh gel should be reapplied to these areas for effective treatment.

Suitable indicators which may be used for this include any indicators which are physiologically acceptable such as, for example, calcium permanganate. Other indicators which are physiologically acceptable will be readily appreciated by a person skilled in the art.

In both of the above options the addition level to the gel would be low, typically less than about 1% w/w, and in some instances may be as low as about 0.01% w/w.

According to a further aspect of the invention, there is provided a sterilisable or sterile gel formulation comprising calcium gluconate as detailed above for use in therapy, particularly for use in the treatment of a burn caused by exposure of a physiological target site to HF.

According to a further aspect of the invention, there is provided method of treating a burn caused by exposure of a physiological target site to HF, comprising the steps of applying to the physiological target site a sterilisable or sterile gel formulation comprising calcium gluconate.

There is also provided a use of a sterile or sterilisable gel formulation comprising calcium gluconate in treating a burn caused by exposure of a physiological target site to HF.

Attempts at producing the gel involved the use of several types of gelling agent in suitable formulations for topical application. In a generic manner the gels contained calcium gluconate, a gelling agent, a pH adjuster and water. These were all subjected to gamma radiation inside a commercial facility.

The invention will now be described further by way of example with reference to the following examples which are intended to be illustrative only and in no way limiting upon the scope of the invention.

### Examples

A number of different gel formulations comprising calcium gluconate have been tested for their stability under gamma irradiation. It was already known that the existing calcium gluconate gel formulation was unstable under gamma irradiation, as there was a significant reduction in the viscosity of the gel, rendering it unfit to be used in the treatment of HF burns. The existing calcium gluconate gel formulation has the following components, excluding small additions of optional components such as preservative and humectants etc. (all amounts quantities are given as w/w):
Natrasol (2.8%)
Calcium gluconate (2.5%)
De-ionised water (to 100%)

The first attempt to modify the gel formulation to impart irradiation stability was to increase the quantity of the gelling agent (Natrasol, a hydroxyethylcellulose-containing product) in the formulation at the expense of a corresponding quantity of de-ionised water. Two samples were submitted for irradiation, having the following respective compositions:
Natrasol (3.0%)
Calcium gluconate (2.5%)
De-ionised water (to 100%)
Natrasol (3.2%)
Calcium gluconate (2.5%)
De-ionised water (to 100%)
Result: FAIL - unacceptable changes in rheology

By "unacceptable changes in rheology" it is intended to mean that the characteristics of the formulation changed significantly due to its exposure to the sterilisation conditions.

Therefore, simply increasing the amount of the existing gelling agent further did not impart gamma irradiation stability to the gel formulation.

The next attempt was to try sodium carboxymethylcellulose as an alternative gelling agent at the same quantity (3.2 wt.%) as the last failed Natrasol sample. One sample was submitted for testing, having the following composition:
Sodium carboxymethylcellulose (3.2%)
Calcium gluconate (2.5%)
De-ionised water (to 100%)
Result: FAIL - unacceptable changes in rheology

Therefore, simply changing the Natrasol existing gelling agent to sodium carboxymethylcellulose did not impart gamma irradiation stability to the gel formulation.

Ammonium hydroxide was added to increase the solubility of the gelling agent. Two further samples were submitted, one with the original gelling agent, Natrasol, and the other with sodium carboxymethylcellulose. The original gelling agent quantity of 2.8 wt. % was used in both samples. The compositions of the formulations were as follows:
Natrasol (2.8%)
Calcium gluconate (2.5%)
De-ionised water (to 100%)
Sodium carboxymethylcellulose (2.8%)
Calcium gluconate (2.5%)
De-ionised water (to 100%)
Result: FAIL - unacceptable changes in rheology

Addition of ammonium hydroxide did not impart gamma irradiation stability to the gel formulation.

A formulation having a different supplier/grade of the original gelling agent, Natrasol, again at 2.8 wt. %, was then submitted:
Hydroxyethylcellulose (2.8%)
Calcium gluconate (2.5%)
De-ionised water (to 100%)
Result: FAIL - unacceptable changes in rheology

Therefore, simply changing the gelling agent for a different grade of hydroxyethylcellulose did not impart gamma irradiation stability to the gel formulation.

It was thought that the calcium gluconate in the formulation may be interacting with the cellulosics. A sample without calcium gluconate having the following composition was therefore submitted (the extra 2.5 wt. % caused by the loss of the calcium gluconate was made up with de-ionised water) to investigate this:
Natrasol (2.8%)
De-ionised water (to 100%)
Result: FAIL - unacceptable changes in rheology

This showed that the calcium gluconate was not directly interacting with the cellulosics and causing the gel structure to fail.

A sample with a different gelling agent again was submitted, this time using xanthan gum, though there are many natural gums that may be used, in the original quantity used for Natrasol in the non-sterile calcium gluconate gel formulation:
Xanthan Gum (2.8%)
Calcium gluconate (2.5%)
De-ionised water (to 100%)
Result: FAIL - unacceptable changes in rheology

A further sample with another different gelling agent was submitted, this time using 3.0 wt. % of carbomer powder:
Carbomer powder (3.0%)
Calcium gluconate (2.5%)
De-ionised water (to 100%)
Result: STABLE

This formulation comprising carbomer powder as the gelling agent was shown to be stable under gamma irradiation and is able to be effectively used in treatment of HF burns. This gelling agent helped to form a sterilisable gel which did not break down during the sterilisation process, which would comply with the new proposed regulations and which can be used on damaged skin in the treatment of burns caused by exposure to HF. Furthermore, only a relatively small amount, 3.0 wt.%, of the gelling agent was required to impart irradiation stability.

The next trials consisted of using autoclave technology to raise the temperature to a sufficiently high level for a prolonged period of time so as to render the microbiological contamination in the gel inactive. A steam autoclave was used as these are the most common types available. A series of gels were made to the formulations above and placed inside sealed packaging, were subjected to a steam sterilisation cycle. Gels produced from the following agents were found to be both sufficiently rheologically stable and microbiologically sterile while still having a freely available calcium content: sodium carboxymethylcellulose, hydroxyethylcellulose , carbomer powder and xanthan gum.

It is of course to be understood that the present invention is not intended to be restricted to the foregoing examples which are described by way of example only.

## Claims

1. A sterile gel formulation comprising calcium gluconate.

2. A gel formulation according to claim 1, wherein the formulation comprises no more than about 7 wt.% of a humectant.

3. A gel formulation according to claim 1 or claim 2, wherein the formulation comprises at least 5 wt.% of free water which is available to form steam under sterilisation conditions.

4. A gel formulation according to any preceding claim, wherein the gel formulation comprises a gelling agent selected from sodium carboxymethylcellulose, hydroxyethylcellulose, carbomer powder and xanthan gum.

5. A gel formulation according to any preceding claim, further comprising a colorant and/or a physiologically acceptable indicator substance.

6. A sterilisable gel formulation comprising calcium gluconate and a carbomer powder, which formulation is stable under gamma-radiation sterilisation conditions.

7. A sterilisable gel formulation according to claim 6, wherein the carbomer powder is present in an amount of between about 1 to about 4 wt.%.

8. A sterilisable gel formulation according to claims 6 or claim 7, wherein the calcium gluconate is present in an amount of between about 1.5 to about 4 wt.%.

9. A sterile gel formulation comprising the formulation of any of claims 6-8 in sterilised form.

10. A sterilisable or sterile gel formulation according to any of claims 1-9 for use in therapy.

11. A sterilisable or sterile gel formulation according to any of claims 1-9 for use in the treatment of a burn caused by exposure of a physiological target site to HF.

12. A method of manufacturing a gel formulation according to any of claims 1-5, comprising the steps of:
i) substantially dissolving a quantity of calcium gluconate in an aqueous solution;
ii) adding a gelling agent to the solution;
iii) mixing the solution until it is substantially homogeneous;
iv) if necessary, adjusting the pH to between about 5.0 and 8.0; and
v) sterilising the gel formulation.

13. A method of treating a burn caused by exposure of a physiological target site to HF, comprising the steps of applying to the physiological target site a sterilisable or sterile gel formulation according to any of claims 1-9.

14. Use of a sterilisable or sterile gel formulation according to any of claims 1-9 in treating a burn caused by exposure of a physiological target site to HF.
